# EUROPEAN PATENT SPECIFICATION

(11) **EP 1 931 370 B1**
(45) Date of publication and mention of the grant of the patent: **08.07.2015**
(21) Application number: 06784276.5
(22) Date of filing: 08.09.2006
(51) Int. Cl.: A61K 38/08, A61P 3/10, A61P 31/12, A61P 31/16, A61P 3/04

(54) **USE OF DES-ASPARTATE-ANGIOTENSIN I**
VERWENDUNG VON DES-ASPARTAT-ANGIOTENSIN I
UTILISATION DE DES-ASPARTATE-ANGIOTENSINE I

(30) Priority: 09.09.2005 US 715156 P
(43) Date of publication of application: 18.06.2008
(73) Proprietor: National University of Singapore, Singapore 119077 (SG)
(72) Inventor: SIM, Meng Kwoon, Singapore 119077 (SG)
(74) Representative: Fleck, Barbara
(86) International application number: PCT/SG2006/000264
(87) International publication number: WO 2007/030082

(56) References cited:
- US-A- 5 773 415
- US-A- 6 100 237
- US-A1- 2003 086 920
- US-B2- 6 589 938
- SIM MENG-KWOON ET AL: "Des-aspartate-angiotensin I exerts hypoglycemic action via glucose transporter-4 translocation in type 2 diabetic KKAy mice and GK rats." ENDOCRINOLOGY DEC 2007 LNKD- PUBMED:17823251, vol. 148, no. 12, December 2007 (2007-12), pages 5925-5932, XP002602742 ISSN: 0013-7227
- SIM M.K. AND YUAN H.T.: 'Effects of des-Asp-angiotensin I on the contractile action of angiotensin II and angiotensin III' EUR. J. PHARMACOL. vol. 278, 1995, pages 175 - 178, XP003009813
- MIN L. ET AL.: 'Effects of des-apo-angiotensin I on angiotensin II-induced incorporation of phenylalanine and thymidine in cultured rat cardiomyocetes and aortic smooth muscle cells' REGULATORY PEPTIDES vol. 95, 2000, pages 93 - 97, XP003009814
- SHURK T. ET AL.: 'Effect of angiotensin peptides on PAI-1 expression and production in human adipocytes' HORMONAL METABOLISM RESEARCH vol. 33, 2001, pages 196 - 200, XP008077751
- DHARMANI M. ET AL.: 'Effect of des-aspartate-angiotensin I on the actions of angiotensin II in the isolated renal and mesenteric vasculature of hypertensive and STZ-induced diabetic rats' REGULATORY PEPTIDES vol. 129, 2005, pages 213 - 219, XP004913564
- KUBA K. ET AL.: 'Angiotensin-converting enzyme 2 in lung diseases' CURRENT OPINION IN PHARMACOLOGY vol. 6, 2006, pages 271 - 276, XP005430485

## Description

### Field of the invention

The present invention relates to the use of des-aspartate-angiotensin I (des-asp-ang I) in the treatment and/or prophylaxis of at least one influenza virus infection or of hyperglycaemia in a subject with obesity, and/or bulimia nervosa.

### Background of the invention

Viral respiratory tract infection is the most common illness in humans and the economic cost for non-influenza-related viral respiratory tract infection approaches $40 billion in the US (Fendrick AM *et al,* 2003), while influenza causes tens of millions of day of restricted activity, bed disability and work loss (Sullivan KM *et al,* 1993). There is also the ever present threat of a pandemic flu outbreak and the apparent unstoppable AIDS virus spreading far and wide. With the ability of viruses to mutate by genetic drift and genetic shift, new viruses that are not susceptible to existing vaccines will continue to come into being. There is, therefore, a need to develop more effective antiviral drugs.

Another medical condition, prolonged or chronic hyperglycaemia, wherein blood glucose levels are elevated from long periods, produces many detrimental effects. This condition is exemplified by diabetes. Diabetes exacts a huge toll in money and human suffering, accounting for more than 100 billion dollars of healthcare costs annually in the United States (DiRamond J, 2003). The number of cases worldwide is estimated at 150 million with an equal number of undiagnosed cases in the First World countries and eight times more undiagnosed cases in the Third World (Levitt NS *et al,* 1993). The disease is characterised by high blood sugar (glucose) resulting from the defects in either insulin secretion or decreased sensitivity of the body's cells to the action of insulin, leading to a condition of hyperglycaemia. Type I diabetes is currently treated by preprandial administration of exogenous insulin and dietary restriction. Current therapy of type 2 diabetes includes lifestyle modifications and the use of a variety of pharmacological agents that aim to increase insulin secretion, decrease hepatic glucose production, and/or increase insulin actions. Despite these approaches, good glycemic control is beyond the reach of most diabetic individuals, and the state of prolonged or chronic hyperglycaemia can cause cardiovascular diseases, stroke, blindness, kidney failure, neurological dysfunction, necrosis and gangrene of extremities. The search for better and more specific agents with physiological properties for hyperglycaemia is highly warranted.

Accordingly, any new and improved treatment of viral infections and/or conditions relating or due to hyperglycaemia will be welcome.

The compound des-aspartate-angiotensin I is an endogenous angiotensin peptide (Sim Mk *et al,* 1994, a,b). It is formed or derived from angiotensin I by a specific aminopeptidase present in blood vessels and the hypothalamus (Sim MK, Qui XS, 1994). Previous studies suggested the use of des-aspartate-angiotensin I in cardiovascular and renal actions (Sim US. 5773415, 6100237, 6589938B2 and US2003/0086920). Sim et al., 1995 ('Effects of des-Asp-angiotensin I on the contractile action of angiotensin II and angiotensin III' EUR. J. PHARMACOL. vol. 278, 1995, pages 175 - 178) relates to des-aspartate-angiotensin I on contractile action of the rabbit aortic ring. Min et al. 2000 ('Effects of des-apo-angiotensin I on angiotensin II-induced incorporation of phenylalanine and thymidine in cultured rat cardiomyocetes and aortic smooth muscle cells' REGULATORY PEPTIDES vol. 95, 2000, pages 93 - 97) relates to incorporation of phenylalanine and thymidine in rat cardiomyocites. However, no further uses are taught or suggested for des-aspartate-angiotensin I.

### Summary of the invention

The present invention addresses the problems above, and in particular, provides new and effective means of treatment and/or prophylaxis of influenza viral infections and hyperglycaemia in a subject with obesity, and/or bulimia nervosa. In particular, the present inventor has surprisingly found that angiotensin I may be used in inducing hypoglycaemia and/or for reducing hyperglycaemia and/or for the treatment of said hyperglycaemia-related conditions as well as in the treatment and/or prophylaxis of influenza viral infections.

Accordingly, in one aspect, the present invention provides des-aspartate-angiotensin I for use in the treatment and/or prophylaxis of at least one influenza viral infection.

In another aspect, there is also provided des-aspartate-angiotensin I for use in the treatment and/or prophylaxis hyperglycaemiain a subject with obesity, and/or bulimia nervosa. It will be appreciated that these hyperglycaemia-related conditions are not a renal-related disorder.

There is also provided the use of des-aspartate-angiotensin I in the preparation of a medicament for the treatment and/or prophylaxis of at least one influenza viral infection; as well as for the treatment and/or prophylaxis of hyperglycaemia in a subject with obesity, and/or bulimia nervosa. Again, it will be appreciated that said conditions are not a renal-related disorder.

Also disclosed are kits for said treatments and/or prophylaxis of said viral infection or said conditions.

Under the relevant aspects above, the at least one influenza viral infection may be influenza virus infection such as an influenza A infection.

The hyperglycaemia-related condition is obesity, and/or bulimia nervosa, although type I diabetes is also disclosed.

The des-aspartate-angiotensin I may be administered in a pharmaceutically and/or therapeutically effective amount. The des-aspartate-angiotensin I may comprise at least one pharmaceutically-acceptable carrier, excipient, diluent and/or adjuvant.

The disclosed kits may further comprise information and/or instructions pertaining to their use.

### Brief description of the figures and tables

### Figures:

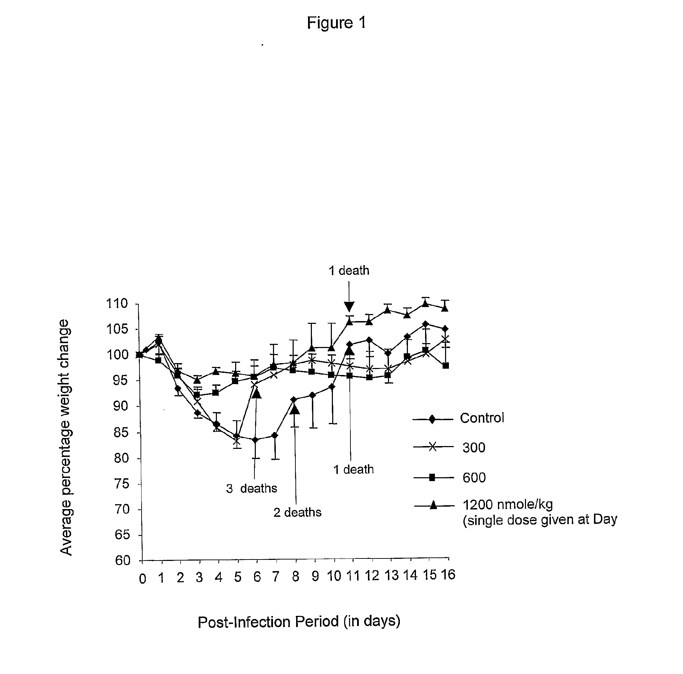

Figure 1 shows the effect of des-aspartate-angiotensin I on weight loss and survival of influenza A virus-infected female BALB/c mice. Six to 7 week-old mice were intranasally infected with 50 µL passage-6 lung homogenate (equivalent to 2.5 x 105 TCID50 of influenza A in MDCK cells). Group of 5-9 mice were then orally administered with one of the following doses of des-aspartate-angiotensin I:- 300 nmole/kg/dat for 8 days (n = 5), 600 nmole/kg/day for 8 days (n = 9), and 120 nmole/kg administered on Day 2 post-infection (n = 9, only one dose was administered). The control group (n = 8) was similarly administered water for 8 days. The weight and survival of the mice were recorded daily for 16 days.

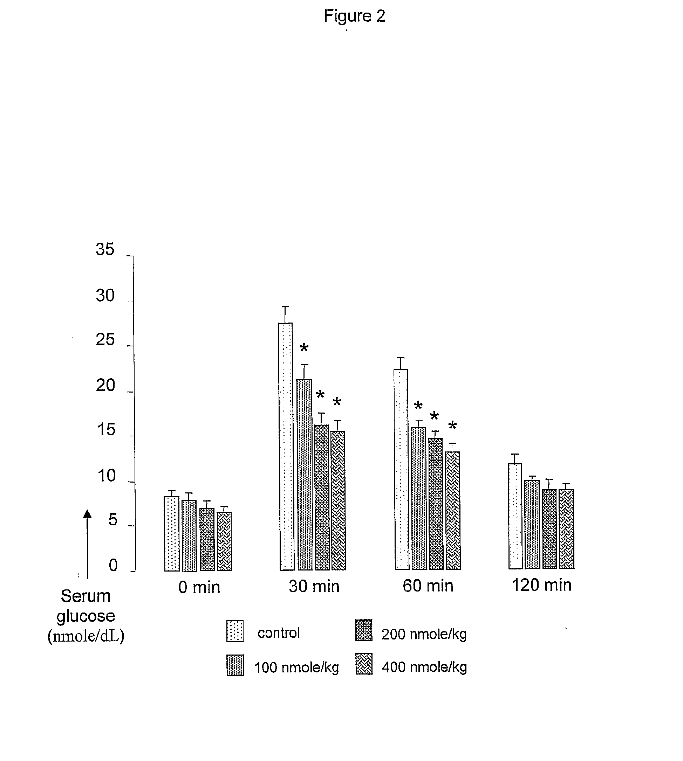

Figures 2 to 6 show the effect of des-aspartate-angiotensin I on blood glucose profile animal models of diabetes.
Figure 2 shows the effect of des-aspartate-angiotensin I on blood glucose profile in diabetic KK-Ay mice. The diabetic KKAy mice were divided into four groups consisting of 6 animals per group. Animals in the control group were intraperitoneally administered 0.1 mL saline. Animals in the second, third, and fourth groups were similarly administered with 100, 200, 400 nmole/kg des-aspartate-angiotensin I, respectively. Treatment with saline and des-aspartate-angiotensin I was carried out daily for 4 weeks. Following this, animals were fasted overnight for 16 hours and oral glucose tolerance test was performed as follows: blood was withdrawn from the orbital sinus for blood glucose determination (time of withdrawal was designated as 0 time), animals were then orally administered glucose (2g/kg), and blood was withdrawn at 30, 60 and 120 min for blood glucose determination. The blood was allowed to clot and blood glucose was measured as serum glucose using a commercial glucose kit from Thermo Electron Corporation, Australia. *Significantly different from the corresponding values of the control untreated mice (p< 0.05, ANOVA followed by post hoc Tukey test).

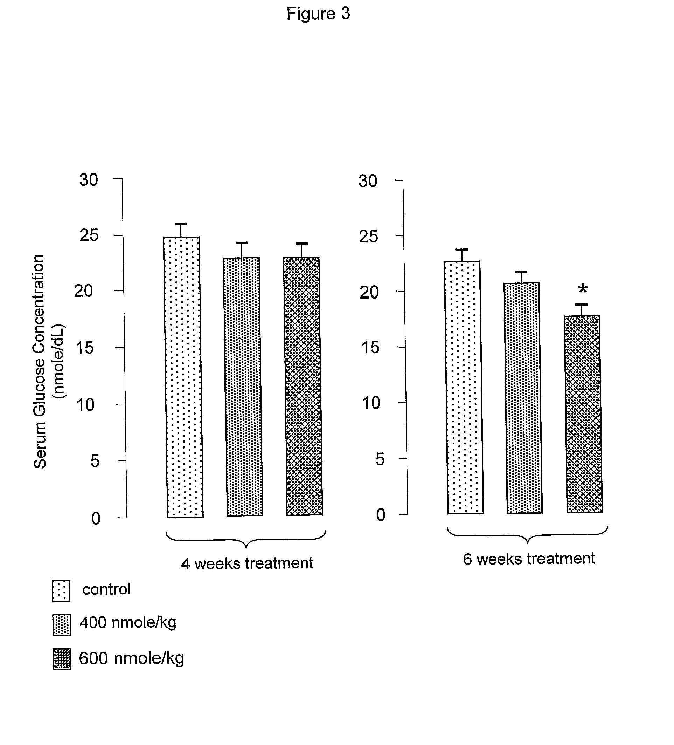

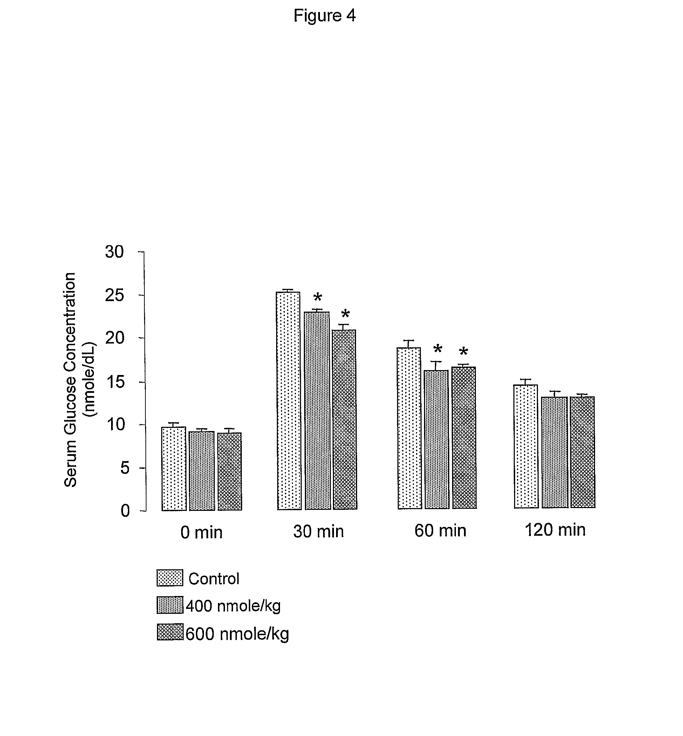

Figure 3 shows the effect of orally administered des-aspartate-angiotensin II on blood glucose profile in diabetic GK rats at 4 and 6 weeks of treatment. The experiment was similarly carried as for KKAy mice. Control animals were administered (by gavage) 0.2 mL of water and groups 1 and 2 animals were similarly administered 400 and 600 nmole/kg des-aspartate-angiotensin I in 0.2 mL water, respectively, for a period 8 weeks. Oral glucose tolerance test (determined at 30 min) was carried out after 4 and 6 weeks of treatment. N = 5 to 6. *Significantly different from the corresponding values of the control untreated rat (p< 0.05, ANOVA followed by post hoc Tukey test). Data obtained after 8 week of treatment are given in another figure, Fig. 4. *Note:* The data show that orally administered DAA-I exerts hypoglycaemic action in diabetic GK rats after 6 weeks of treatment.
Figure 4 shows the effect of orally administered des-aspartate-angiotensin I on blood glucose profile in diabetic GK rats at 8 weeks of treatment. This figure gives the data of a full oral glucose tolerance test conducted at 8 weeks of the same experiment described in Fig. 3. *Significantly different from the value of the corresponding control (p<0.05, ANOVA post hoc Tukey test).

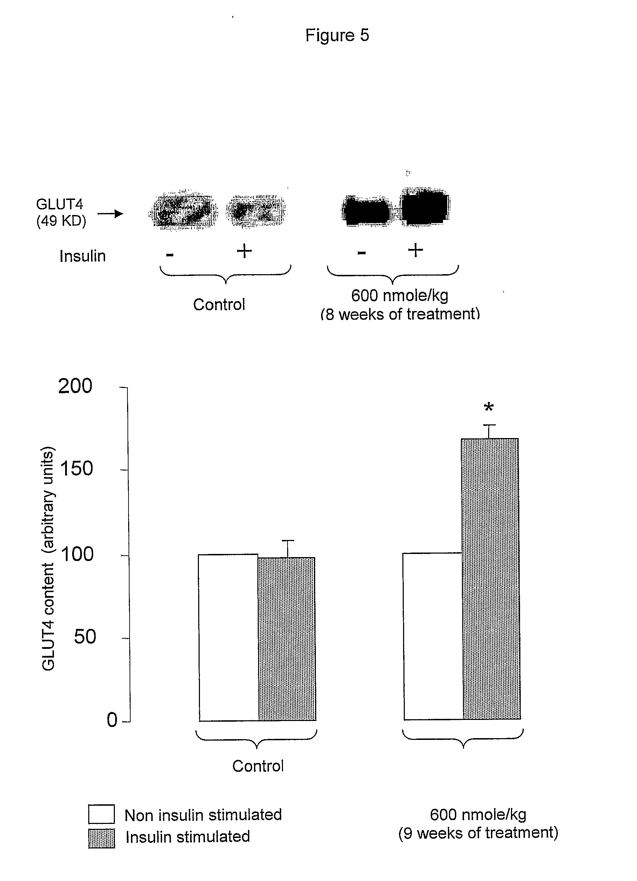

Figure 5 shows the Effects of des-aspartate-angiotesnin I on insulin-induced translocation of GLUT-4 in skeletal muscle of diabetic GK rats. Upper panel: Representative Western blot of plasma membrane GLUT4 in skeletal muscle of des-aspartate-angiotensin I treated and non treated diabetic GK rats. Lower panel: Relative (to the non insulin stimulated samples) levels of plasma membrane GLUT4 in skeletal muscle of des-aspartate-angiotensin I treated and control diabetic GK rats. N =3 for each histogram. *Significantly different from the value of the corresponding control (p<0.05, ANOVA post hoc Tukey test).

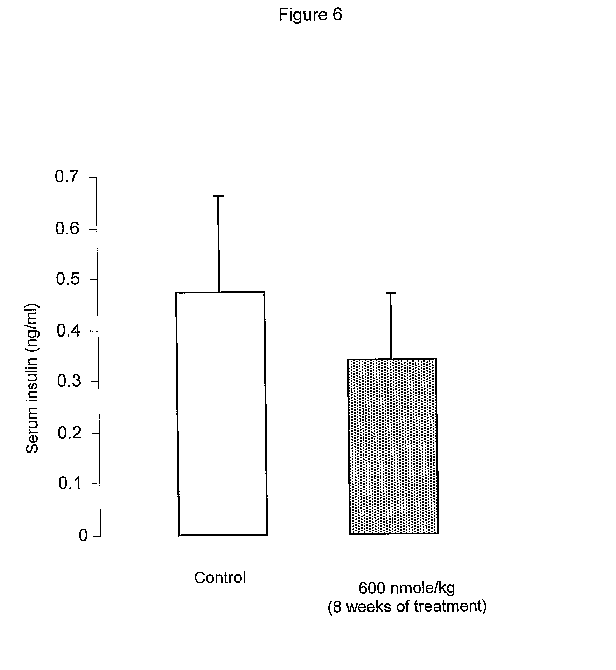

Figure 6 shows the effect of des-aspartate-angiotesnin I on serum insulin level of diabetic GK rats. Serum from 8 weeks treated animals (as described in EXAMPLE 9) were assayed for insulin concentration by the "Ultra Senstive Rat Insulin ELISA kit' (Crystal Chem Inc., IL, USA). The values were from serum taken at 30 min of the oral glucose tolerance test. There were no significant difference between the values of the control and treated group.

### Tables:

Table 1 shows some examples of unnatural amino acids.
Tables 2 and 3 show the effect of des-aspartate-angiotensin I in animal models of influenza.
Table 2 shows the percentage weight change and survival of influenza A virus-infected BALB/c mice treated with orally-administered des-aspartate-angiotensin I
Table 3 shows the percentage weight change and survival of influenza A virus-infected BALB/c mice treated with intraperitoneally-administered des-aspartate-angiotensin I
Tables 4 and 5 show the effects of des-aspartate-angiotensin I in control of blood glucose levels in animal models of diabetes.
Table 4 shows the data of the oral glucose tolerance test carried out in diabetic KKAy mice that were treated with intraperitoneally-administered des-aspartate-angiotensin I
Table 5 shows data of oral the glucose tolerance test carried out in diabetic KKAy mice that were treated with orally-administered des-aspartate-angiotensin I.

### Detailed description of the invention

Bibliographic references mentioned in the present specification are for convenience listed in the form of a list of references and added at the end of the examples.

Des-aspartate angiotensin I have been previously described for use in treatment of certain disorders. For example, the effect of des-aspartate angiotensin I in segmental glomerulosclerosis rat model resembling renal lesions in humans arising from various disorders including those arising from diabetus mellitus was previously determined (US2003/0086920).

However, the prior art does not teach or suggest use of des-aspartate-angiotensin I for the treatment and/or prophylaxis of viral infections and/or for the induction of hypoglycaemia and/or reduction of hyperglycaemia-related conditions. In particular, the present invention relates to the use of desasparatate-angiotensin I in the treatment of hyperglycaemia-related condition(s), excluding renal-related disorders. In particular, the condition(s) treated according to the present invention do not include renal-related disorders described in US2003/0086920. Indeed, said conditions are limited to hyperglycaemia in a subject with obesity, and/or bulimia nervosa.

The present invention relates to the use of des-aspartate-angiotensin I for the treatment and/or prophylaxis of influenza viral infections, preferably influenza A infections.

While researching the effects of des-aspartate-angiotensin I on the cardiopulmonary functions of influenza A virus-infected mice, it was surprisingly discovered that des-aspartate-angiotensin I increased the survival of the animals.

Similarly, it was surprisingly discovered that des-aspartate-angiotensin I exerts marked hypoglycaemic effects in animal models of hyperglycaemia. Accordingly, the present disclosure provides new treatments, prophylaxes and/or pharmaceutical compositions for influenza viral infections and/or the hyperglycaemia-related conditions of hyperglycaemia in a subject with obesity, and/or bulimia nervosa using des-aspartate-angiotensin I.

### Treatment of viral infections

The present disclosure provides a method for the treatment and/or prophylaxis of at least one influenza viral infection and its symptoms comprising administering to a subject des-aspartate-angiotensin I. In particular, the des-aspartate-angiotensin I may be administered in a therapeutically and/or pharmaceutically effective amount. More in particular, the method may comprise administering to a subject an therapeutically and/or pharmaceutically effective amount of des-aspartate-angiotensin I, for a time and under conditions sufficient for the onset of the influenza viral infection and/or its symptoms to be prevented, inhibited and/or delayed or the symptoms of the viral infection to be ameliorated. Derivatives of angiotensin I are disclosed which may be des-aspartate-angiotensin I, or a derivative, homologue and/or analogue thereof.

The at least one influenza viral infection may be a an influenza A infection.

Induction of hypoglycaemia/reduction of hyperglycaemia

Also disclosed is a method of inducing hypoglycaemia and/or reducing hyperglycaemia in a subject, the method comprising administering to the subject des-aspartate-angiotensin I, its derivative, a functional part and/or analogue thereof.

In particular, disclosed is a method of treatment and/or prophylaxis of at least one hyperglycaemia-related condition comprising administering to a subject des-aspartate-angiotensin I, its derivative, a functional part and/or analogue thereof, wherein the at least one hyperglycaemia-related condition excludes renal-related disorders.

In particular, disclosed is also a method for the treatment and/or prophylaxis of at least one of the following states or conditions: hyperglycaemia, particularly prolonged or uncontrolled hyperglycaemic conditions as in type I diabetes, obesity, bulimia nervosa excluding renal disorders arising from hyperglycaemia as in Type 2 diabetes (diabetes mellitus), the method comprising administering to a subject at least one derivative of angiotensin I. In particular, the at least one derivative of angiotensin I is des-aspartate-angiotensin I, its derivative, a functional part and/or analogue thereof. In particular, the angiotensin I derivative is administered for a time and under conditions sufficient for the condition and/or its symptoms to be prevented, inhibited, delayed and/or ameliorated.

The present disclosure provides des-aspartate-angiotensin I, preferably a pharmaceutical composition thereof, for use in the treatment and/or prophylaxis of hyperglycaemia in prolonged or uncontrolled hyperglycaemic conditions such as in a subject with obesity and/or bulimia nervosa. Excluded are renal disorders arising from hyperglycaemia as in Type 2 diabetes mellitus. The pharmaceutical composition may comprise, optionally, a pharmaceutically acceptable carrier, excipient, adjuvant and/or diluent.

The compositions may be administered in a therapeutically and/or pharmaceutically effective dose, and/or for a time and under conditions sufficient for its symptoms to be prevented, inhibited or delayed and/or ameliorated.

Throughout this specification, unless the context requires otherwise, the word "comprise", or variations such as "comprises" or "comprising, will be understood to imply the inclusion of a stated element or integer or group of elements or integers but not the exclusion of any other element or integer or group of elements or integers.

An "effective amount" refers to an amount effective, at dosages and for periods of time necessary to achieve the desired therapeutic result, such as to prevent, inhibit and/or delay the onset and/or ameliorate the symptoms of the particular hyperglycaemia-related disorders of hyperglycaemia in a subject with obesity, and/or bulimia nervosa. While the effective amount may vary according to various factors such as the disease state, age, sex, and weight of the individual. The disclosed derivatives of des-aspartate-angiotensin I includes any mutant, fragment, part and/or portion of angiotensin I or des-aspartate-angiotensin I including molecules comprising single or multiple amino acid substitutions, deletions and/or additions to angiotensin I or to des-aspartate-angiotensin I. The processes of substitutions, deletions and/or additions may result in a derivative or peptide that has more amino acids than the original angiotensin I or des-aspartate-angiotensin I. The processes of substitutions, deletions and/or additions may result in a derivative or peptide that has more amino acids than the original angiotensin I or des-aspartate angiotensin I

The preferred derivative disclosed is des-aspartate-angiotensin I or a derivative, homologue and/or analogue thereof. Reference to a homologue and/or an analogue disclosed herein includes a mimotope or peptide and/or analogue mimetic. Such derivatives, homologues and/or analogues may function in place of des-aspartate-angiotensin I and/or its equivalent or they may act as an agonist thereof or, when necessary, an antagonist thereof.

As stated above, a derivative disclosed herein includes single or multiple amino acid substitutions, additions and/or deletions to des-aspartate-angiotensin I and/or angiotensin I. The final derivative disclosed is a peptide having similar functions and consists of no less than two amino acid sequence of the original angiotensin I or des-aspartate-angiotensin.

Amino acid insertional derivatives of des-Asp-angiotensin I disclosed herein include amino and/or carboxyl terminal fusions as well as intra-sequence insertions of single or multiple amino acids. Insertional amino acid sequence variants disclosed herein are those in which one or more amino acid residues are introduced into a predetermined site in the des-aspartate-angiotensin I although random insertion is also possible with suitable screening of the resulting product.

Deletional variants disclosed herein are characterized by the removal of one or more amino acids from the sequence. Substitutional amino acid variants are those in which at least one residue in the sequence has been removed and a different residue inserted in its place.

Where the des-aspartate-angiotensin I disclosed herein is derivatized by amino acid substitution, the amino acids are generally replaced by other amino acids having like properties, such as hydrophobicity, hydrophilicity, electronegativity, bulky side chains and the like. Amino acid substitutions are either of single or multiple residues. Amino acid insertions will usually be in the order of about 1-7 amino acid residues and deletions will range from about 1-7 residues.Deletions or insertions may be made in adjacent pairs, i.e. a deletion of two residues or insertion of two residues.

Homologues disclosed herein include functionally, structurally or stereochemically similar polypeptides from, for example, other sources such as for livestock animals, laboratory test animals or primates. The similar peptides may also be homologues of des-aspartate-angiotensin I.

Analogues and mimetics disclosed herein include molecules which contain non-naturally occurring amino acids as well as molecules which do not contain amino acids but nevertheless behave functionally the same as the des-aspartate-angiotensin I. Analogues disclosed herein include modifications to side chains, incorporation of unnatural amino acids and/or their derivatives during peptide synthesis and the use of crosslinkers and other methods which impose conformational constraints on the peptide molecule or their analogues.

Examples disclosed herein of incorporating unnatural amino acids and derivatives during peptide synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, ornithine, sarcosine, 4-amino-3-hydroxy-6-methylheptanoic acid, 2-thienyl alanine and/or D-isomers of amino acids.

It is also disclosed that crosslinkers can be used, for example, to stabilize 3D conformations, using homo-bifunctional crosslinkers such as the bifunctional imido esters having (CH.2)n spacer groups with n=1 to n=6, glutaraldehyde, N-hydroxysuccinimide esters and heterobifunctional reagents which usually contain an aminoreactive moiety such as N-hydroxysuccinimide and another group specific-reactive moiety.

It is disclosed that all these types of modifications may be important to stabilize the subject des-Asp-angiotensin I. This may be important if used, for example, in the manufacture of a vaccine or therapeutic composition or in detection assays. Examples of unnatural amino acids are presented in Table 1.

**Table 1**

| **Non-conventional amino acid** | **Code** | **Non-conventional amino acid** | **Code** |
|---|---|---|---|
| α-aminobutyric acid | Abu | L-N-methylalanine | Nmala |
| α-amino-α-methylbutyrate | Mgabu | L-N-methylarginine | Nmarg |
| aminocyclopropane-carboxylate | Cpro | L-N-methylasparagine | Nmasn |
| | | L-N-methylaspartic acid | Nmasp |
| aminoisobutyric acid | Aib | L-N-methylcysteine | Nmcys |
| aminonorbornyl-carboxylate | Norb | L-N-methylglutamine | Nmgln |
| | | L-N-methylglutamic acid | Nmglu |
| cyclohexylalanine | Chexa | L-N-methylhistidine | Nmhis |
| cyclopentylalanine | Cpen | L-N-methylisolleucine | Nmile |
| D-alanine | Dal | L-N-methylleucine | Nmleu |
| D-arginine | Darg | L-N-methyllysine | Nmlys |
| D-aspartic acid | Dasp | L-N-methylmethionine | Nmmet |
| D-cysteine | Dcys | L-N-methylnorleucine | Nmnle |
| D-glutamine | Dgln | L-N-methylnorvaline | Nmnva |
| D-glutamic acid | Dglu | L-N-methylornithine | Nmorn |
| D-histidine | Dhis | L-N-methylphenylalanine | Nmphe |
| D-isoleucine | Dile | L-N-methylproline | Nmpro |
| D-leucine | Dleu | L-N-methylserine | Nmser |
| D-lysine | Dlys | L-N-methylthreonine | Nmthr |
| D-methionine | Dmet | L-N-methyltryptophan | Nmtrp |
| D-ornithine | Dorn | L-N-methyltyrosine | Nmtyr |
| D-phenylalanine | Dphe | L-N-methylvaline | Nmval |
| D-proline | Dpro | L-N-methylethylglycine | Nmetg |
| D-serine | Dser | L-N-methyl-t-butylglycine | Nmtbug |
| D-threonine | Dthr | L-norleucine | Nle |
| D-tryptophan | Dtrp | L-norvaline | Nva |
| D-tyrosine | Dtyr | α-methyl-aminoisobutyrate | Maib |
| D-valine | Dval | α-methyl-α-aminobutyrate | Mgabu |
| D-α-methylalanine | Dmala | α-methylcyclohexylalanine | Mchexa |
| D-α-methylarginine | Dmarg | α-methylcylcopentylalanine | Mcpen |
| D-α -methylasparagine | Dmasn | α-methyl-α-napthylalanine | Manap |
| D-α-methylaspartate | Dmasp | α-methylpenicillamine | Mpen |
| D-α -methylcysteine | Dmcys | N-(4-aminobutyl)glycine | Nglu |
| D-α-methylglutamine | Dmgln | N-(2-aminoethyl)glycine | Naeg |
| D-α-methylhistidine | Dmhis | N-(3-aminopropyl)glycine | Norn |
| D-α -methylisoleucine | Dmile | N-amino-α-methylbutyrate | Nmaabu |
| D-α-methylleucine | Dmleu | α-napthylalanine | Anap |
| D-α-methyllysine | Dmlys | N-benzylglycine | Nphe |
| D-α-methylmethionine | Dmmet | N-(2-carbamylethyl)glycine | Ngln |
| D-α-methylornithine | Dmorn | N-(carbamylmethyl)glycine | Nasn |
| D-α-methylphenylalanine | Dmphe | N-(2-carboxyethyl)glycine | Nglu |
| D-α-methylproline | Dmpro | N-(carboxymethyl)glycine | Nasp |
| D-α-methylserine | Dmser | N-cyclobutylglycine | Ncbut |
| D-α-methylthreonine | Dmthr | N-cycloheptylglycine | Nchep |
| D-α-methyltryptophan | Dmtrp | N-cyclohexylglycine | Nchex |
| D-α-methyltyrosine | Dmty | N-cyclodecylglycine | Ncdec |
| D-α-methylvaline | Dmval | N-cylcododecylglycine | Ncdod |
| D-N-methylalanine | Dnmala | N-cyclooctylglycine | Ncoct |
| D-N-methylarginine | Dnmarg | N-cyclopropylglycine | Ncpro |
| D-N-methylasparagine | Dnmasn | N-cycloundecylglycine | Ncund |
| D-N-methylaspartate | Dnmasp | N-(2,2-diphenylethyl)glycine | Nbhm |
| D-N-methylcysteine | Dnmcys | N-(3,3-diphenylpropyl)glycine | Nbhe |
| D-N-methylglutamine | Dnmgln | N-(3-guanidinopropyl)glycine | Narg |
| D-N-methylglutamate | Dnmglu | N-(1-hydroxyethyl)glycine | Nthr |
| D-N-methylhistidine | Dnmhis | N-(hydroxyethyl))glycine | Nser |
| D-N-methylisoleucine | Dnmile | N-(imidazolylethyl))glycine | Nhis |
| D-N-methylleucine | Dnmleu | N-(3-indolylyethyl)glycine | Nhtrp |
| D-N-methyllysine | Dnmlys | N-methyl-α-aminobutyrate | Nmgabu |
| N-methylcyclohexylalanine | Nmchexa | D-N-methylmethionine | Dnmmet |
| D-N-methylornithine | Dnmorn | N-methylcyclopentylalanine | Nmcpen |
| N-methylglycine | Nala | D-N-methylphenylalanine | Dnmphe |
| N-methylaminoisobutyrate | Nmaib | D-N-methylproline | Dnmpro |
| N-(1-methylpropyl)glycine | Nile | D-N-methylserine | Dnmser |
| N-(2-methylpropyl)glycine | Nleu | D-N-methylthreonine | Dnmthr |
| D-N-methyltryptophan | Dnmtrp | N-(1-methylethyl)glycine | Nval |
| D-N-methyltyrosine | Dnmtyr | N-methyla-napthylalanine | Nmanap |
| D-N-methylvaline | Dnmval | N-methylpenicillamine | Nmpen |
| α-aminobutyric acid | Gabu | N-(p-hydroxyphenyl)glycine | Nhtyr |
| L-t-butylglycine | Tbug | N-(thiomethyl)glycine | Ncys |
| L-ethylglycine | Etg | penicillamine | Pen |
| L-homophenylalanine | Hphe | L-α-methylalanine | Mala |
| L- -methylarginine | Marg | L-α-methylasparagine | Masn |
| L- -methylaspartate | Masp | L-α-methyl-t-butylglycine | Mtbug |
| L- -methylcysteine | Mcys | L-methylethylglycine | Metg |
| L--methylglutamine | Mgln | L-α-methylglutamate | Mglu |
| L- -methylhistidine | Mhis | L-α-methylhomophenylalanine | Mhphe |
| L- -methylisoleucine | Mile | N-(2-methylthioethyl)glycine | Nmet |
| L- -methylleucine | Mleu | L-α-methyllysine | Mlys |
| L- -methylmethionine | Mmet | L-α-methylnorleucine | Mnle |
| L- -methylnorvaline | Mnva | L-α-methylornithine | Morn |
| L- -methylphenylalanine | Mphe | L-α-methylproline | Mpro |
| L- -methylserine | Mser | L-α-methylthreonine | Mthr |
| L- -methyltryptophan | Mtrp | L-α-methyltyrosine | Mtyr |
| L- -methylvaline | Mval | L-N-methylhomophenylalanine | Nmhphe |
| N-(N-(2,2-diphenylethyl) | Nnbhm | N-(N-(3,3-diphenylpropyl) | Nnbhe |
| carbamylmethyl)glycine | | carbamylmethyl)glycine | |
| 1-carboxy-1-(2,2-diphenyl-ethylamino)cyclopropane | Nmbc | | |

Having now generally described the invention, the same will be more readily understood through reference to the following examples which are provided by way of illustration, and are not intended to be limiting to the present invention.

### EXAMPLES

Standard molecular biology techniques known in the art and not specifically described were generally followed as described in Sambrook and Russel, Molecular Cloning: A Laboratory Manual, Cold Springs Harbor Laboratory, New York (2001).

### Example 1 - Source of Materials

Des-aspartate-angiotensin was purchased from Bachem (Dubendorf, Switzerland). Des-aspartate-angiotensin I can be prepared by techniques well known in the art. Influenza A/Aichi/2/68 virus and Madin-Darby Canine Kidney cells were purchased from ATCC. The virus has a titer of 106.75 CEID50/0.2 ml in 2 days on 11 day old SPF CE. Six to 7 weeks old female BALB/c mice were obtained from the Animal Center, National University of Singapore. Six to 7 weeks old diabetic KKAy mice were purchased from CLEA, Japan.

### Example 2 - Development of a Mouse Model for Influenza A Virus

Two BALB/c mice were intranasally infected with 50 µL of influenza A virus. Two days later, the infected mice were sacrificed by cervical dislocation and their lungs were removed. 0.3 g of lung tissue was homogenized in 1 mL of PBS containing 1000 units/L penicillin and 10 µg/L streptomycin. The lung homogenate was cleared of connective tissue by centrifuging at 5,000 rpm for 5 minutes on a bench-top centrifuge. This homogenate was labeled as passage-1 lung homogenate. A second batch of 2 BALB/c mice was infected with 50 µL of passage-1 lung homogenate. The process of passaging was repeated and the virus virulence and titer in each passage of lung homogenate was monitored by assaying its infectivity in Madin-Darby Canine Kidney (MDCK) cells, which is a host cell for influenza A virus. The virulence and titer of the influenza A virus increased progressively with each passage. Fifty µL of passage-6 lung homogenate, equivalent to 2.5 x 105 TCID50 of the virus in MDCK cells, were then used to infect 6-7 weeks old female BALB/c mice for the study of the antiviral action of des-aspartate-angiotensin I.

### Example 3 - Monitoring and Treatment (with Orally-Administerd Des-aspartate-angiotensin I) of Influenza A Virus-Infected BALB/c Mice

BALB/c mice when infected with passage-6 lung homogenate lost weight and suffered fatality. The following protocol described by Johasson et al was used to study the effect of des-aspartate-angiotensin on these parameters. Six to 7 weeks old female BALB/c mice were intranasally infected with 50 µL passage-6 lung homogenate. Groups of 5-9 mice were orally administered with one of the following dose of des-aspartate-angiotensin I:- 300 nmole/kg daily for 8 days, 600 nmole/kg daily for 8 days, and 1200 nmole/kg on day 2 post virus infection (i.e. only one dose was administered). A group of 8 mice were similarly administered water for 8 days and served as the control group. The weight and survival of the mice were recorded daily for 16 days.

### Example 4 - Effect of Orally-Administered Des-aspartate-angiotensin I on Virus-Infected BALB/c Mice

The results of the study are summarized in Table 2 and depicted in Fig. 1. The daily weight of each surviving animal was expressed as a percentage of its weight at Day 0 (i.e. day of virus inoculation). The data of the control and each treatment group were expressed as the mean ± SEM. Significant differences were determined by one way ANOVA and post hoc Tukey test. The accepted level of significance was p < 0.05. Des-aspartate-angiotensin I is an antiviral agent in term of its ability to attenuate the weight loss and survival of the virus-infected animals. Effective antiviral activity was observed with a dose of 600 nmole/kg administered daily for 8 days, and a single dose of 1200 nmole/kg given on Day 2.

### Example 5 - Monitoring and Treatment (with Intraperitoneally-administered Desaspartate-angiotensin I) of Influenza A Virus-infected BALB/c Mice

Thirty BALB/c mice were infected with 55 µL passage-6 lung homogenate and the weight of each animal was monitored daily as described in EXAMPLES 3 and 4. Animals were intraperitoneally administered with either 60 nmole/kg des-aspartate-angiotensin I in 0.1 ml of phosphate buffer saline (PBS) or 0.1 ml PBS on the day when their weight loss was 15% or greater during the first post-infection week. This treatment was continued for 7 days. Of the 30 animals, 14 animals lost more than 15% of their weight during the first post-infection week. Hence, 7 animals were randomly chosen for des-aspartate-angiotensin I treatment and 7 animals for vehicle treatment. The weight of the treated animals were monitored till either the animal died or regained 100% of their initial weight. Surviving animals were euthanized and their lung homogenate was prepared and assayed for influenza A virus as described in Example 2.

**Table 2 *Significantly different from the corresponding control value (p < 0.05, ANOVA post hoc Tukey test) Bold numbers within brackets indicate number of animals that died on the day of recording. ^{#}Average value from two surviving mice i.e. no SEM.**

| Post Infection Period (in Days) | Average Percentage Weight Change (weight of individual animals at Day 0 was taken as 100%) | | | |
|---|---|---|---|---|
| | Control (n =8) | 300 nmole/kg/day for 16 days (n = 5) | 600 nmole/kg/day for 16 days (n = 9) | 1200 nmole/kg given as a single dose on Day 2 (n = 9) |
| 0 | 100 | 100 | 100 | 100 |
| 1 | 102.4 ± 2.2 | 102.1 ± 1.5 | 98.7 ± 1.3 | 103.5 ± 0.5 |
| 2 | 93.4 ± 1.4 | 96.2 ± 2.0 | 95.8 ± 1.4 | 96.8 ± 0.6 |
| 3 | 88.7 ± 1.0 | 90.8 ± 2.4 | 92.0 ± 1.7 | **95.1 ± 0.8*** |
| 4 | 86.4 ± 1.3 | 85.8 ± 2.9 | **92.4 ± 1.8*** | **96.7 ± 1.2*** |
| 5 | 84.1 ± 2.3 | 83.2 ± 3.4 | **94.8 ± 1.8*** | **96.3 ± 2.1*** |
| 6 | 83.3 ± 3.6 | 94.1^{#} **(3)** | **95.5 ± 2.1*** | **95.7 ± 3.1*** |
| 7 | 84.1 ± 4.6 | 95.7^{#} | **97.8 ± 2.6*** | **97.8 ± 4.0"** |
| 8 | 91.0 ± 5.4 **(2)** | 97.9^{#} | 96.7 ± 2.9 | 98.3 ± 4.3 |
| 9 | 91.8 ± 6.2 | 98.7^{#} | 96.3 ± 3.5 | 101.1 ± 4.7 |
| 10 | 93.4 ± 7.1 | 98.1^{#} | 95.6 ± 4.0 | 101.1 ± 4.8 |
| 11 | 101.6 ± 2.8 **(1)** | 97.4^{#} | 95.4 ± 4.5 | 105.9 ± 1.3 **(1)** |
| 12 | 102.4 ± 3.1 | 96.9^{#} | 95.1 ± 5.0 | 106.1 ± 1.3 |
| 13 | 99.9 ± 5.8 | 96.8^{#} | 95.5 ± 5.2 | 108.1 ± 1.2 |
| 14 | 103.0 ± 3.9 | 98.2^{#} | 99.1 ± 3.4 | 107.2 ± 1.3 |
| 15 | 105.4 ± 3.8 | 99.6^{#} | 100.3 ± 4.2 | 109.4 ± 1.2 |
| 16 | 104.4 ± 3.5 | 102.4^{#} | 97.0 ± 4.8 | 108.4 ± 1.5 |

### Example 6 - Effect of Intraperitoneally-administered Des-aspartate-angiotensin I on Virus-infected BALB/c Mice

The data of the study are summarized in Table 3. The weight of each animal was expressed as a percentage of its weight at Day 0. All mice that were treated with saline died within a week. In the des-aspartate-angiotensin I treated mice, only 1 mouse died. Six animals survived and regained their original weight. No influenza A virus was detected in the lung homogenate of the surviving mice. The results show that des-aspartate-angiotensin I exerts effective antiviral action in preventing influenza A virus-infected mice from dying.

### Example 7 - Effect of Intraperitoneally-Administered Des-aspartate-angiotensin I on Blood Glucose Profile in Diabetic KKAy Mice

The diabteic KKAy mice were divided into four groups consisting of 6 animals per group. Animals in the control group were intraperitoneally administered 0.1 mL saline. Animals in the second, third, and fourth groups were similarly administered with 100, 200, 400 nmole/kg des-aspartate-angiotensin I, respectively in 0.1 ml of PBS. Treatment with saline and des-aspartate-angiotensin I was carried out daily for 4 weeks. Following this, animals were fasted overnight for 16 hours and oral glucose tolerance test was performed as follows: blood was withdrawn from the orbital sinus for blood glucose determination (time of withdrawal was designated as 0 time), animals were then orally administered glucose (2g/kg), and blood was withdrawn at 30, 60 and 120 min for blood glucose determination. The blood was allowed to clot and blood glucose was measured as serum glucose using a commercial glucose kit from Thermo Electron Corporation, Australia. Table 3 shows that des-aspartate-angiotensin I, administered at doses of 200 and 400 nmole/kg/day for 4 weeks, significantly lowered the blood glucose level at 30 and 60 min of the oral glucose tolerance test. The data, graphically displayed in Fig. 2, show that des-aspartate-angiotensin I possesses hypoglycaemic action and can be use to treat the symptoms of hyperglycaemia.

### Example 8 - Dose-Range Finder Experiment for Orally Administered Desaspartate-angiotensin I

A dose-range finder experiment to determine the hypoglycaemic dose for orally administered des-aspartate-angiotensin I was also carried out. In this experiment, the animals in each of the three treatment groups of 7 diabetic KKAy mice were orally administered (by gavage) 200, 400, and 600 nmole/kg/day des-aspartate-angiotensin I in volume of 0.1 mL, respectively. Animals in the control group were similarly administered 0.1 mL water. The treatment period was 4 weeks. Oral glucose tolerance test was carried out as described in Example 5, except that blood was sampled at 0 and 30 min. The data, presented in Table 4, show that des-aspartate-angiotensin I is an effective oral hyperglycemic agent at an oral dose of 600 nmole/kg.

### Example 9 - Effect of Orally-Administered Des-aspartate-angiotensin I on Blood Glucose Profile in Diabetic KKAy Mice

In this experiment, the animals in each of the three treatment groups of 7 diabetic KKAy mice were orally administered (by gavage) 200, 400, and 600 nmole/kg/day des-aspartate-angiotensin I in volume of 0.1 mL water, respectively. Animals in the control group were similarly administered 0.1 mL water. The treatment period was 4 weeks. Oral glucose tolerance test was carried out as described in EXAMPLE 7, except that blood was sampled at 0 and 30 min. The data, presented in Table 5, show that des-aspartate-angiotensin I exerts significant hypoglycaemic action at an oral dose of 600 nmole/kg.

### Example 10 - Effect of Orally-administered Des-aspartate-anaiotensin I on Blood Glucose Profile in Diabetic GK Rats

Diabetic GK rats were divided into three groups, consisting of six animals in each group. Animals in groups 1 and 2 were administered, by gavage, one of the following: 400 or 600 nmole/kg of des-aspartate-angiotensin I in a volume of 0.2 mL water daily for up to 8 weeks. Control animals in group 3 were similarly administered 0.2 mL water. Thirty min oral glucose tolerance test was performed fortnightly as a spot test to detect significant hypoglycaemic action (if any) that arose from des-aspartate-angiotensin I treatment. As shown in Fig. 3, significant hypoglycaemic action was detected at 6 weeks of treatment. Animals were treated for a further 2 weeks and a full oral glucose tolerance test was performed thereafter. The data, presented in Fig. 4, show that the hypoglycaemic action of des-aspartate-angiotensin I was also significantly expressed at 8 weeks of treatment. The results of this study show that des-aspartate-angiotensin I is an effective oral hypoglycaemic agent and can be used to treat the hyperglycemia in diabetes.

### Example 11 - Effect of Orally-Administered Des-aspartate-angiotensin I on Insulin-Induced Translocation of Glucose Transporter-4 (GLU4) to Plasma Membrane in Skeletal Muscles of Diabetic GK Rats

The diabetic GK rats described in EXAMPLE 9 were similarly treated for another week. At 9 weeks of treatment, animals were fasted overnight and half the animals in each group were intraperitoneally administered with PBS and the other half with insulin (0.5 U/ kg). After 30 minutes, the animals were sacrificed by cervical dislocation. Hind limb skeletal muscles were rapidly excised and frozen in liquid nitrogen. Muscle samples were stored at -80 oC until used. The hindlimb muscles were subsequently thawed over an ice pack and plasma membranes were prepared as described by Dombrowaski et al . Thirty µg of protein from the 25 % interface of the discontinued sucrose gradient was subjected to SDS-PAGE, transferred to PVDF sheets and incubated with anti-GLUT4 polyclonal primary antibody (1: 800), washed and followed by incubation with anti-goat secondary antibody (1: 10,000). The bands were detected at 49 kDa. The data, presented in Fig. 5, show that des-aspartate-angiotensin I significantly enhances the insulin-induced translocation of GLUT4 to the plasma membrane in skeletal muscle of diabetic GK rats. As insulin resistance is the hallmark of diabetes, it is concluded that the des-aspartate-angiotensin I exerts marked hypoglycaemic action in diabetic GK rats by overcoming insulin resistance in these animals. This is especially so as serum level of insulin was not significantly affected in the same diabetic GK rats that were treated with 600 nmole/kg of des-aspartate-angiotensin I (see Fig. 6 for details).

| Post Infection Period (in Days) | Table 3: Percentage Weight Change of Individual Mouse | | | | | | | | | | | | | |
|---|---|---|---|---|---|---|---|---|---|---|---|---|---|---|
| | Saline treated mice | | | | | | | Des-aspartate-angiotensin I treated mice | | | | | | |
| | 1 | 2 | 3 | 4 | 5 | 6 | 7 | 1 | 2 | 3 | 4 | 5 | 6 | 7 |
| 0 | 100.0 | 100.0 | 100.0 | 100.0 | 100.0 | 100.0 | 100.0 | 100.0 | 100.0 | 100.0 | 100.0 | 100.0 | 100.0 | 100.0 |
| 1 | 99.4 | 98.1 | 98.7 | 99.2 | 99.5 | 99.3 | 94.8 | 99.4 | 100.4 | 99.0 | 98.4 | 99.3 | 98.5 | 98.9 |
| 2 | 96.5 | 97.1 | 97.5 | 97.9 | 98.2 | 96.6 | 87.8 | 99.3 | 99.8 | 96.3 | 97.1 | 96.6 | 97.3 | 95.8 |
| 3 | 89.8 | 96.8 | 96.4 | 96.8 | 95.8 | 93.9 | 86.0 | 99.4 | 99.0 | 90.3 | 94.6 | 88.6 | 94.7 | 93.9 |
| 4 | 88.2 | 93.2 | 93.1 | 86.0 | 85.7 | 87.5 | **81.5** | 97.5 | 96.6 | 85.7 | 86.2 | **83.3** | 92.1 | 90.8 |
| 5 | 87.7 | 86.5 | 90.9 | **80.7** | **82.0** | **84.7** | **80.1** | 90.3 | 91.7 | **82.8** | 85.7 | **81.0** | 90.1 | 89.0 |
| 6 | 81.8 | **82.9** | 86.7 | **76.8** | **78.7** | **83.4** | **78.6** | 87.5 | 87.4 | **80.8** | **82.7** | **78.2** | **85.2** | 88.8 |
| 7 | **80.9** | **81.1** | **84.1** | **69.8** | **77.4** | **78.4** | **73.9** | **85.0** | **83.8** | **80.7** | **76.8** | **73.9** | **82.8** | **84.4** |
| 8 | **74.6** | **79.4** | **81.5** | **65.4** | **74.9** | **76.3** | **69.4** | **86.2** | **80.4** | **79.7** | **72.9** | **71.2** | **83.2** | **82.4** |
| 9 | **71.8** | **75.3** | **80.1** | died | **72.7** | **74.0** | **65.1** | **90.3** | **78.8** | **81.4** | **69.9** | **71.8** | **80.5** | **78.6** |
| 10 | **70.8** | **70.6** | **76.4** | | **69.4** | **71.0** | **61.0** | **93.0** | **77.5** | **80.5** | **69.0** | **73.2** | **82.3** | **77.7** |
| 11 | **69.6** | **died** | **72.6** | | **68.2** | **68.9** | died | **99.3** | **77.1** | **82.1** | **67.7** | 75.8 | **88.9** | **74.5** |
| 12 | **68.8** | | died | | died | **died** | | **101.2** | **78.9** | 80.0 | **64.9** | 80.1 | **88.9** | **74.5** |
| 13 | died | | | | | | | | **80.8** | 76.6 | 63.0 | 82.8 | 97.4 | **72.3** |
| 14 | | | | | | | | | 82.6 | 75.2 | 61.3 | 86.3 | 102.8 | 74.0 |
| 15 | | | | | | | | | 84.3 | 83.0 | 58.9 | 90.1 | | 76.7 |
| 16 | | | | | | | | | 89.4 | 90.3 | 58.9 | 94.6 | | 78.6 |
| 17 | | | | | | | | | 94.8 | 93.5 | 58.7 | 99.3 | | 81.2 |
| 18 | | | | | | | | | 98.7 | 99.1 | died | 101.0 | | 83.2 |
| 19 | | | | | | | | | 100.9 | 100.8 | | | | 86.8 |
| 20 | | | | | | | | | | | | | | 91.8 |
| 21 | | | | | | | | | | | | | | 95.7 |
| 22 | | | | | | | | | | | | | | 98.4 |
| 23 | | | | | | | | | | | | | | 100.1 |

**Table 4**

| Dose of des-aspartate angiotens in I | Serum glucose level at different sampling time (nmole/dL) | | | |
|---|---|---|---|---|
| | 0 min | 30 min | 60 min | 120 min |
| Control | 8.3 ± 0.6 | 27.3 ± 1.9 | 22.2 ± 1.3 | 12.2 ± 1.3 |
| 100 nmole/kg | 7.9 ± 0.9 | **21.2 ± 1.6*** | **13.4 ± 1.3*** | 9.9 ± 0.5 |
| 200 nmole/kg | 6.9 ± 0.9 | **16.0 ± 1.4*** | **14.5 ± 0.9*** | 8.7 ± 1.2 |
| 400 nmole/kg | 6.5 ± 0.6 | **15.4 ± 1.6*** | **13.0 ± 1.1*** | 8.8 ± 0.6 |

| | | | | |
|---|---|---|---|---|
| *Significantly different from the corresponding value of the Control (p < 0.05, ANOVA post hoc Tukey test) | | | | |

**Table 5**

| Dose of desaspartate-angiotensin I | Serum glucose level at different sampling time (nmole/dL) | |
|---|---|---|
| | 0 min | 30 min |
| Control | 9.0 ± 1.0 | 33.7 ± 1.7 |
| 200 nmole/kg | 8.9 ± 1.0 | 37.7 ± 2.6 |
| 400 nmole/kg | 9.1 ± 1.6 | 26.0 ± 3.6 |
| 600 nmole/kg | 8.9 ± 0.7 | **17.5 ± 1.9*** |

| | | |
|---|---|---|
| *Significantly different from the corresponding value of the Control (p < 0.05, ANOVA post hoc Tukey test) | | |

### References

DiRamond J. The double puzzle of diabetes. Nature 2003; 423: 599-602.
Dombrowski et al A new procedure for the isolation of plasma membranes, T tubules, and internal membranes from skeletal muscle. Am J Physiol 1996; 270:E667-E676.
Fendrick et al. The economic burden of non-influenza-related viral respiratory tract infection in the United States. Arch Intern Med 2003; 163:487-494.
Levitt NS et al. The prevalence and identification of risk factors for NIDDM in urban Africans in Cape Town, South Africa. Diabetes Care 1993; 16:601-607.
Johasson et al. Infection-permissive immunization with influenza virus neuraminidase prevents weight loss in infected mice. Vaccine 1993; 11:1037-1039.
Sim MK et al. Degradation of angiotensin I in the endothelium and smooth muscle of the rat aorta. Biochem Pharmacol 1994(a); 45:1524-1527.
Sim MK et al. Degradation of angiotensin I to [des-Asp1]angiotensin I by a novel aminopeptidase in the rat hypothalamus. Biochem Pharmacol 1994(b); 48:1043-1046.
Sim MK, Qui XS. Angiotensins in plasma of hypertensive rats and human. Regul Peptides 2003; 111:179-182.
Sullivan KM, et al. Estimates of the US health impact of influenza. Am J Public Health 1993; 83:1712-1716.
US 5,773,416
US 6,100,237
US 6, 589, 938 B2
US 2003/0086920

## Claims

1. Des-aspartate-angiotensin I for use in the treatment and/or prophylaxis of hyperglycaemia in a subject with obesity, and/or bulimia nervosa.

2. Des-aspartate-angiotensin I for use in the treatment and/or prophylaxis of at least one influenza virus infection.

3. Des-aspartate-angiotensin I according to claim 2, wherein the influenza virus infection is influenza A infection.

4. Use of des-aspartate-angiotensin I for the preparation of a medicament for the treatment and/or prophylaxis of hyperglycaemia in a subject with obesity, and/or bulimia nervosa.

5. Use of des-aspartate-angiotensin I for the preparation of a medicament for the treatment and/or prophylaxis of at least one influenza virus infection.

6. The use according to claim 5, wherein the influenza virus infection is influenza A infection.

7. The use according to any one of claims 4 to 6, wherein the medicament, further comprises at least one pharmaceutically acceptable carrier, excipient, diluent and/or adjuvant.

## Patentansprüche

1. Des-Aspartat-Angiotensin I zur Verwendung bei der Behandlung und/oder Prophylaxe von Hyperglykämie bei einem Lebewesen mit Adipositas, und/oder Bulimie.

2. Des-Aspartat-Angiotensin I zur Verwendung bei der Behandlung und/oder Prophylaxe von wenigstens einer Influenzavirus-Infektion.

3. Des-Aspartat-Angiotensin I nach Anspruch 2, wobei die Influenzavirus-Infektion Influenza A-Infektion ist.

4. Verwendung von Des-Aspartat-Angiotensin I für die Herstellung eines Medikamentes für die Behandlung und/oder Prophylaxe von Hyperglykämie bei einem Lebewesen mit Adipositas, und/oder Bulimie.

5. Verwendung von Des-Aspartat-Angiotensin I für die Herstellung eines Medikamentes für die Behandlung und/oder Prophylaxe von wenigstens einer Influenzavirus-Infektion.

6. Verwendung nach Anspruch 5, wobei die Influenzavirus-Infektion Influenza A-Infektion ist.

7. Verwendung nach einem der Ansprüche 4 bis 6, wobei das Medikament weiter wenigstens einen pharmazeutisch akzeptablen Träger, ein Verdünnungsmittel und/oder ein Adjuvans umfasst.

## Revendications

1. Des-aspartate-angiotensine I pour une utilisation dans le traitement et/ou la prophylaxie de l'hyperglycémie chez un sujet souffrant d'une obésité, et/ou d'une boulimie nerveuse.

2. Des-aspartate-angiotensine I pour une utilisation dans le traitement et/ou la prophylaxie d'au moins une infection par le virus de la grippe.

3. Des-aspartate-angiotensine I selon la revendication 2, où l'infection par le virus de la grippe est une infection par le virus de la grippe A.

4. Utilisation de des-aspartate-angiotensine I pour la préparation d'un médicament pour le traitement et/ou la prophylaxie de l'hyperglycémie chez un sujet souffrant d'une obésité, et/ou d'une boulimie nerveuse.

5. Utilisation de des-aspartate-angiotensine I pour la préparation d'un médicament pour le traitement et/ou la prophylaxie d'au moins une infection par le virus de la grippe.

6. Utilisation selon la revendication 5, dans laquelle l'infection par le virus de la grippe est une infection par le virus de la grippe A.

7. Utilisation selon l'une quelconque des revendications 4 à 6, dans laquelle le médicament comprend en outre au moins un support, un excipient, un diluant et/ou un adjuvant pharmaceutiquement acceptable(s).
